# EUROPÄISCHE PATENTSCHRIFT

(11) **EP 1 073 620 B1**
(45) Veröffentlichungstag und Bekanntmachung des Hinweises auf die Patenterteilung: **09.07.2003**
(21) Anmeldenummer: 99920693.1
(22) Anmeldetag: 16.04.1999
(51) Int. Cl.: C07C 51/16, C07C 67/03, C07C 69/60, C07C 29/17, C07D 307/08, C07D 307/32

(54) **VERFAHREN ZUR HERSTELLUNG VON 1,4-BUTANDIOL**
METHOD FOR PRODUCING 1,4-BUTANEDIOL
PROCEDE POUR LA PRODUCTION DE 1,4-BUTANEDIOL

(30) Priorität: 23.04.1998 DE 19818248
(43) Veröffentlichungstag der Anmeldung: 07.02.2001
(73) Patentinhaber: BASF AKTIENGESELLSCHAFT, 67056 Ludwigshafen (DE)
(72) Erfinder: FISCHER, Rolf, D-69121 Heidelberg (DE); KAIBEL, Gerd, D-68623 Lampertheim (DE); PINKOS, Rolf, D-67098 Bad Dürkheim (DE); RAHN, Ralf-Thomas, D-68167 Mannheim (DE)
(74) Vertreter: Isenbruck, Günter, Dr.
(86) Internationale Anmeldenummer: EP9902587
(87) Internationale Veröffentlichungsnummer: WO99055659

(56) Entgegenhaltungen:
- WO-A-97/43242
- DE-A- 2 543 673
- DE-A- 2 553 959

## Beschreibung

Die Erfindung betrifft ein Verfahren zur Herstellung von 1,4-Butandiol sowie gegebenenfalls Tetrahydrofuran (THF) und γ-Butyrolacton (GBL) aus Oxidationsabgasen von Butan bzw. Benzol durch Absorption von Maleinsäureanhydrid (MSA) mit hochsiedenden Lösungsmitteln, Veresterung des Maleinsäureanhydrids mit einem Alkohol zum Diester und katalytische Hydrierung des Maleinsäurediesters.

1,4-Butandiol ist ein wichtiges Ausgangsprodukt zur Herstellung von Polyestern wie Polybutylenterephthalat, sowie von γ-Butyrolacton und THF.
Daher sind zahlreiche Verfahren zur Herstellung von 1,4-Butandiol, sowie Gemischen, die 1,4-Butandiol enthalten, aus MSA, erhalten durch katalytische Oxidation von Kohlenwasserstoffen, bekannt.

In DE-2543673 und DE-2553959 wird 1,4-Butandiol durch Katalytische Hydrogenierung von MSA (estern) beschrieben.

So kann MSA durch Gasphasenoxidation von Benzol oder n-Butan hergestellt werden. Der gasförmige Oxidationsaustrag enthält im allgemeinen neben MSA Wasser, Sauerstoff, Stickstoff, Kohlendioxid, nicht umgesetztes Benzol bzw. Butan sowie geringe Mengen an Ameisensäure, Essigsäure, Acrylsäure und Propionsäure. Daneben sind Oxidationsprodukte der Verunreinigungen der Oxidationsedukte wie i-Butan enthalten. Dabei handelt es sich z.B. um Citraconsäureanhydrid, Furan, Acrolein, Crotonaldehyd, Crotonsäure und Methacrolein.

Da in der industriellen Praxis im allgemeinen 1,4-Butandiol-Reinheiten von über 99,9 % gefragt sind, wurde das MSA bisher, um solche Reinheiten zu erhalten, in den meisten Fällen gereinigt, bevor es z.B. in Form der Ester zu 1,4-Butandiol hydriert wurde.

Die WO 97/43242 beschreibt ein Verfahren zur Herstellung von 1,4-Butandiol, γ-Butyrolacton und THF ohne Vorreinigung des aus Benzol, gemischten C₄-olefinischen Verbindungen oder n-Butan durch katalytische Oxidation gewonnenen Maleinsäureanhydrids.

In diesem Verfahren wird MSA aus einem MSA enthaltenden Dampfstrom mit einem organischen Lösungsmittel, das um mindestens 30°C höher siedet als der Maleinsäurediester, in einer Absorptionszone in Kontakt gebracht. Es wird ein Abgasstrom zurückgehalten, und das MSA in dem hochsiedenden Lösungsmittel wird in einer Veresterungszone unter Veresterungsbedingungen mit einem geeigneten C₁- bis C₄-Alkohol zum Diester umgesetzt. Der erhaltene Diester wird mit einem Wasserstoffstrom aus dem organischen Lösungsmittel herausgestrippt und in der Gasphase an einem heterogenen Hydrierkatalysator hydriert. Aus dem Hydrieraustrag können die Wertprodukte 1,4-Butandiol, γ-Butyrolacton und THF gewonnen werden.

Dieses Verfahren beeinhaltet einige Nachteile. Bei der Strippung des Maleinsäurediesters aus dem hochsiedenden Lösungsmittel werden, neben dem Maleinsäurediester, leichter siedende Verbindungen aus Verunreinigungen des Maleinsäureanhydrids und Spuren des Lösungsmittels mit herausgestrippt. Diese gelangen in die Hydrierphase, so daß nach der Hydrierung ein erhöhter Trennaufwand nötig ist.

Desweiteren muß die Strippung bei demselben Druck wie die Gasphasenhydrierung durchgeführt werden. Das bedeutet, daß ein hoher Wasserstoffstrom im Kreis geführt werden muß (im Beispiel der WO 97/43242: 320 Mol Wasserstoff auf ein Mol Diester). Die auftretenden hohen Stripptemperaturen können dazu führen, daß Crack-Prozesse ablaufen.

Weitere Nachteile treten bei der Gasphasenhydrierung auf. So muß die Temperatur des Gasstroms oberhalb des Taupunktes des Maliensäurediesters gehalten werden. Dazu ist ein hoher Wasserstoffstrom notwendig und über die Rückführung des überschüssigen Wasserstoffs fallen hohe Energiekosten an. Die kurze Verweilzeit während der Gasphasenhydrierung führt zur Bildung von Nebenprodukten und damit zu einer Ausbeuteverminderung und einem erhöhten Trennaufwand bei der Destillation. Bei der Gasphasenhydrierung von Maleinsäurediestern sind nur geringe Katalysatorbelastungen möglich, da sonst die adiabatische Temperaturerhöhung über den Reaktor zu groß wird, was zu einer Abnahme der Selektivität führt. Eine Gegensteuerung durch Erhöhung der Wasserstoffmenge ist möglich, jedoch wegen hoher Kreisgaskosten unwirtschaftlich.

Aufgabe der vorliegenden Erfindung ist daher ein Verfahren zur Herstellung von 1,4-Butandiol und gegebenenfalls γ-Butyrolacton und THF, in dem 1,4-Butandiol mit guter Ausbeute und in hoher Reinheit ohne vorhergehende Reinigung des Maleinsäureanhydrids erhalten werden kann.

Eine weitere Aufgabe ist die Bereitstellung eines einfachen kostengünstigen Verfahrens zur Herstellung von 1,4-Butandiol und gegebenenfalls γ-Butyrolacton und THF.

Diese Aufgaben werden durch ein Verfahren zur Herstellung von 1,4-Butandiol und gegebenenfalls γ-Butyrolacton und THF gelöst, durch Oxidation von n-Butan oder Benzol zu einem Maleinsäureanhydrid enhaltenden Produktstrom, Absorption von Maleinsäureanhydrid aus dem Produktstrom mit einem hochsiedenden inerten Lösungsmittel in einer Absorptionsstufe, wobei ein flüssiger Absorptionsaustrag erhalten wird, Veresterung des flüssigen Absorptionsaustrages mit einem C₁- bis C₅-Veresterungsalkohol in einer Veresterungsstufe zu einem Veresterungaustrag, der den entsprechenden Diester und hochsiedendes inertes Lösungmittel enthält, anschließende Hydrierung des Veresterungsaustrages, wobei ein Hydrieraustrag erhalten wird, der die Wertprodukte 1,4-Butandiol und gegebenenfalls γ-Butyrolacton und THF enthält, welcher destillativ in die Wertprodukte und den Veresterungsalkohol aufgetrennt wird und der Veresterungalkohol in die Veresterungszone zurückgeführt wird. Das erfindungsgemäße Verfahren ist dann dadurch gekennzeichnet, daß der Veresterungsaustrag vor der Hydrierung unter vermindertem Druck destillativ in den Diester und das inerte Lösungsmittel getrennt wird, das inerte Lösungsmittel in die Absorptionsstufe zurückgeführt wird und der Diester in der Flüssigphase an einem Festbettkatalysator hydriert wird.

Durch dieses Verfahren können die Wertprodukte 1,4-Butandiol und gegebenenfalls Y-Butyrolacton und THF in wenigen einfachen Verfahrensschritten kostengünstig und in guter Ausbeute aus Maleinsäureanhydrid gewonnen werden. Durch die destillative Reinigung des Veresterungaustrages vor der Hydrierung können die Wertprodukte nach der Hydrierung in hoher Reinheit erhalten werden. Die Flüssigphasenhydrierung ermöglicht im Vergleich zur Gasphasenhydrierung hohe Katalysatorbelastungen, was die Wirtschaftlichkeit des erfindungsgemäßen Verfahrens weiter erhöht.

Durch das erfindungsgemäße Verfahren kann nach der Destillation des Hydrieraustrages 1,4-Butandiol in einer Reinheit von über 99,9 mol-%, bevorzugt von mindestens 99,95 mol-%, erhalten werden.

Das gewonnenene hochreine 1,4-Butandiol eignet sich hervorragend als Ausgangssubstanz zur Herstellung von Polyestern wie Polybutylenterephthalat sowie zur Herstellung von γ-Butyrolacton und THF.

Die Herstellung des Maleinsäureanhydrids erfolgt durch Oxidation von Benzol oder n-Butan im allgemeinen in der Gasphase an einem Katalysator auf der Basis von Vanadiumoxiden. Die Herstellung kann genauso oder in ähnlicher Weise erfolgen wie in WO 97/43242 beschrieben. In der partiellen Oxidation von Benzol wird üblicherweise ein geträgerter, mit z.B. MoO₃-aktivierter Vanadiumpentoxid-Katalysator eingesetzt.
Die Reaktionstemperatur beträgt von 400°C bis 455°C und der Reaktionsdruck von etwa 1 bar bis etwa 3 bar. Es wird die etwa vierfache Menge der theoretisch benötigten Luftmenge verwendet, um die Explosionsgrenzen nicht zu überschreiten. Die Kontaktzeit beträgt circa 0,1 Sekunden.

Wenn n-Butan als Ausgangsstoff eingesetzt wird, wird üblicherweise Vanadiumpentoxid als Katalysator bei einer Temperatur von etwa 350°C bis etwa 450°C und einem Druck von etwa 1 bar bis etwa 3 bar verwendet. Das Luft/n-Butan-Verhältnis kann, bei Verwendung eines geeigneten Reaktors, bei etwa 20:1 liegen, auch wenn das Mischungsverhältnis an sich zu einem leicht entzündlichen Gemisch führt.

Man erhält einen gasförmigen Oxidationsaustrag, der im allgemeinen neben Maleinsäureanhydrid Wasser, Sauerstoff, Stickstoff, Kohlendioxid, unumgesetztes Benzol bzw. n-Butan sowie kleinere Mengen organischer Verunreinigungen wie Ameisensäure, Essigsäure, Acrylsäure und Propionsäure enthält. Desweiteren können Oxidationsprodukte von Verunreinigungen der Ausgangsstoffe im Oxidationsaustrag enthalten sein.

Die Absorption des durch Oxidation von Benzol bzw n-Butan in dem gasförmigen Oxidationsaustrag enthaltenden Maleinsäureanhydrids kann, genauso oder ähnlich wie in WO 97/43242 beschrieben, mit einem hochsiedenden inerten organischen Lösungsmittel in einer Absorptionsstufe erfolgen. Dazu wird der Maleinsäureanhydrid enthaltende gasförmige Oxidationsaustrag bei einer Temperatur von circa 60°C bis 160°C und einem Druck von circa 1 bar bis 3 bar mit einem hochsiedenden Lösungsmittel in Kontakt gebracht, wobei ein flüssiger Absorptionsaustrag erhalten wird, der Maleinsäureanhydrid in dem hochsiedenden Lösungsmittel enthält.

Die Absorption von Maleinsäureanhydrid durch das hochsiedende organische Lösungsmittel kann dabei durch Durchblasen eines gasförmigen maleinsäureanhydridhaltigen Stroms durch das Lösungsmittel erfolgen. Alternativ kann das Lösungsmittel in den maleinsäureanhydridhaltigen Dampf gesprüht werden oder die Absorption im Gegenstromverfahren durchgeführt werden.

Bevorzugt eingesetzte Losungmittel sind solche, deren Siedepunkte mindestens 10°C über dem des dem Veresterungsalkohol entsprechenden Maleinsäurediesters liegen. Das Losungsmittel sollte gegenüber Maleinsäureanhydrid und dem entsprechenden Diester unter herrschenden Reaktionsbedingungen inert und weitgehend wasserunlöslich und/oder unfähig, Wasser aufzunehmen sein Beispiele für geeignete hochsiedende organische Lösungsmittel sind in WO 97/43242 aufgelistet.

Geeignete Lösungsmittel sind beispielsweise Dibutylphthalat, Tricresylphosphat, Dibutylmaleat, hochmolekulare Wachse, aromatische Lösungsmittel mit einem Molekulargewicht zwischen 150 und 400 und einem Siedepunkt von über 140°C, wie Dibenzylbenzol und Dialkylphthalatester. Wenn Ester eingesetzt werden, ist es bevorzugt, daß die Alkyleinheit dieser Ester der des eingesetzten Veresterungsalkohols entspricht, um Umesterungen zu vermeiden. Wenn Methanol als Veresterungsalkohol eingesetzt wurde, ist es daher bevorzugt, als hochsiedende Ester z.B. Dimethylphthalat, Dimethyl-2,3-naphthalindicarboxylat, Dimethylcyclohexandicarboxylat oder Methylester langkettiger Fettsäuren einzusetzen. Es können auch hochsiedende Ether, wie Tetraethylenglycoldimethylether, eingesetzt werden.

Der Absorptionsaustrag, der Maleinsäureanhydrid und das hochsiedende inerte Lösungsmittel enthält, wird in einer Veresterungsstufe mit einem C₁- bis C₅-Monoalkohol zur Veresterung des Maleinsäureanhydrids zu Maleinsäurediester genauso oder ähnlich wie in WO 97/43242 beschrieben, umgesetzt. Im allgemeinen werden als Veresterungsalkohole Methanol, Ethanol, n-Propanol, n-Butanol, i-Butanol, n-Pentanol oder i-Pentanol eingesetzt. Bevorzugt sind Methanol und Ethanol. Die Veresterung des Maleinsäureanhydrids mit Alkohol kann ein- oder mehrstufig mit oder ohne Katalysator in dem hochsiedenden Lösungsmittel erfolgen.

Die nicht-katalytische Reaktion kann bei Einsatz von hochsiedenden Alkoholen bei erhöhter Temperatur unter kontinuierlicher Entfernung des Reaktionswassers erfolgen.

Die katalytische Veresterung von Maleinsäureanhydrid kann mit festen, sauren Katalysatoren erfolgen. Es können auch homogen lösliche Katalysatoren wie Schwefelsäure, Phosphorsäure, p-Toluolsulfonsäure, Heteropolysäuren oder Lewissäuren wie Zinnoleat oder Tetraalkoxytitanat eingesetzt werden.

Die Veresterungstemperaturen sind abhängig vom eingesetzten Alkohol, und die Veresterung wird unter Eigendruck durchgeführt.

Diese können mit dem organischen Lösungsmittel zusammen in die Absorptionsstufe zurückgeführt werden, sofern das Lösungsmittel höher siedet als der Maleinsäurediester, und sind in der anschließenden Veresterungsstufe wieder aktiv.

Die Abtrennung des überschüssigen Alkohols und des Reaktionswassers, sowie des Wassers, das über die Absorptionsstufe in die Veresterungsstufe gelangte, kann während oder nach der Veresterung erfolgen. Vor der Rückführung des Alkohols in die Veresterungsstufe wird dieser vom Wasser befreit, welches ausgeschleust wird.

Der erhaltene Veresterungsaustrag wird unter verminderten Druck destillativ in den Diester und das hochsiedende Lösungsmittel getrennt.

Die Destillation wird bei einem Druck von im allgemeinen 30 bis 800 mbar, bevorzugt 50 bis 200 mbar, in einer Destillationskolonne durchgeführt. Die Sumpftemperatur beträgt im allgemeinen 100 bis 250°C, bevorzugt 120 bis 190°C, besonders bevorzugt 125 bis 180°C.

In einer bevorzugten Kolonnenschaltung, wenn das Lösungsmittel gegenüber dem Diester hochsiedend ist, ist der Zulauf des zu destillierenden Gemisches oberhalb des Sumpfes des Destillationskolonne. Die Niedersieder, z.B. restliches Wasser und der Veresterungsalkohol zusammen mit dem Diester, werden über den Kopf der Kolonne abdestilliert. Im Sumpf der Kolonne sammelt sich das hochsiedende organische Lösungsmittel, gegebenenfalls zusammen mit dem Veresterungskatalysator.

Noch vorteilhafter ist es, den Diester ohne weitere Niedersieder wie restliches Wasser und Veresterungsalkohol, zu gewinnen. Dazu wird der Diester über einen Seitenarm im Verstärkungsteil der Kolonne entnommen.

Der auf diese Weise gewonnene Diester wird in der Flüssigphase an einem heterogenen Hydrierkatalysator bei erhöhter Temperatur und erhöhtem Druck hydriert. Die Hydrierung erfolgt in einem Reaktor oder mehreren hintereinander geschalteten Reaktoren.

Die Hydriertemperatur beträgt im allgemeinen 60 bis 300°C, bevorzugt 70 bis 250°C, besonders bevorzugt 80 bis 240°C. Bei niedrigen Temperaturen wird bevorzugt die C-C-Doppelbindung hydriert, bei höheren Temperaturen bevorzugt die Esterbindung.

Die Hydrierung wird bei einem Druck von im allgemeinen 30 bar bis 330 bar, bevorzugt 50 bar bis 300 bar, besonders bevorzugt 100 bar bis 290 bar bei einer Verweilzeit von 0,5 bis 2 Stunden durchgeführt. Die C-C-Doppelbindung kann bereits bei niedrigem Druck, gegebenenfalls separat, hydriert werden.

Die Belastung des Hydrierkatalysators beträgt im allgemeinen 0,2 bis 1,3 kg Diester/Liter Katalysatorvolumen h, bevorzugt 0,3 bis 1 kg, besonders bevorzugt 0,35 bis 0,8 kg.

Zur Abführung der Hydrierwärme wird die Hydrierung von energiereichen Edukten in der Flüssigphase üblicherweise, zumindest im Hauptreaktor, mit Produktrückführung durchgeführt. Dabei liegt das Gewichtsverhältnis zwischen dem Umlauf (Rückführung) und dem Zulauf im allgemeinen zwischen 1 und 100, bevorzugt zwischen 2 und 50, besonders bevorzugt zwischen 3 und 25.

Das molare Verhältnis von Frischwasserstoff zu Diester liegt im allgemeinen zwischen 5 und 8, bevorzugt zwischen 5,01 und 7, besonders bevorzugt zwischen 5,02 und 6.

Als Hydrierkatalysatoren werden im allgemeinen heterogene, zur Hydrierung von Carbonylgruppen geeignete Katalysatoren eingesetzt. Es können sogenannte Vollkatalysatoren, katalytisch wirkende Metalle ohne Trägermaterial, oder Trägerkatalysatoren (Katalysatoren, die ein Trägermaterial enthalten) verwendet werden. Bevorzugt werden Trägerkatalysatoren eingesetzt.

Beispiele für geeignete Hydrierkatalysatoren sind im Houben-Weyl, Methoden der organischen Chemie, Band IV/Ic, S. 16 bis 26, Georg Thieme Verlag, 1980 beschrieben.

Bevorzugt werden in dem erfindungsgemäßen Verfahren Hydrierkatalysatoren eingesetzt, deren katalytisch aktive Komponente ein oder mehrere Elemente aus den Gruppen Ib, VIb, VIIb, VIIIb sowie IIIa, IVa, Va des Periodensystems der Elemente enthalten. Von diesen werden Kupfer, Chrom, Rhenium, Kobalt, Rhodium, Nickel, Palladium, Eisen, Platin, Indium, Zinn, Antimon bevorzugt eingesetzt. Besonders bevorzugt sind Kupfer, Kobalt, Palladium, Platin und Rhodium, ganz besonders bevorzugt ist Kupfer.

Beispiele für geeignete Vollkatalysatoren sind Raney-Katalysatoren auf der Basis von Nickel, Kupfer, Kobalt; Palladium-Schwarz, Platin-Schwarz (wie sie im Houben-Weyl definiert sind), Kupfer-Schwamm, Legierungen oder Mischungen aus z.B Palladium/Rhenium, Platin/Rhenium, Palladium/Nickel, Palladium/Kobalt, Palladium/Rhenium/Silber.

In dem erfindungsgemäßen Verfahren können auch Fällungskatalysatoren eingesetzt werden. Die Herstellung der Fällungskatalysatoren erfolgt durch Ausfällung der katalytisch aktiven Komponente aus ihren Salzlösungen, insbesondere aus Lösungen der Nitrate und/oder Acetate, z.B. durch Zugabe von Alkalimetall-Lösungen und/oder Erdalkalihydroxid- und/oder -carbonatlösungen. So können die katalytisch aktiven Komponenten als z.B. schwerlösliche Hydroxide, Oxidhydrate, basische Salze oder Carbonate ausgefällt werden.

Die Niederschläge werden anschließend getrocknet und durch Kalzinierung bei im allgemeinen 300 bis 700°C, bevorzugt 400 bis 600°C in die entsprechenden Oxide, Mischoxide und/oder gemischt-valentigen Oxide umgewandelt

Die calzinierten oxidischen Verbindungen werden durch Behandlung mit Wasserstoff oder wasserstoffenthaltenden Gasen im allgemeinen bei 50 bis 700°C, bevorzugt bei 100 bis 400°C zu den betreffenden Metallen und/oder oxidischen Verbindungen niederer Oxidationsstufen reduziert und so in die eigentliche katalytisch wirksame Form überführt. Dabei wird solange reduziert, bis kein Wasser mehr gebildet wird.

Die Fällungskatalysatoren können Trägermaterial enthalten. Dazu wird die katalytisch aktive Komponente in Gegenwart des betreffenden Trägermaterials gefällt. In einem bevorzugten Verfahren werden die katalytisch aktive Komponente und das Trägermaterial gleichzeitig aus den betreffenden Salzlösungen gefällt.

Neben den oben genannten Fällungskatalysatoren, die als Trägerkatalysatoren eingesetzt werden können, sind auch Trägerkatalysatoren geeignet, bei denen die katalytisch aktiven Komponenten auf andere Art auf das Trägermaterial aufgebracht worden sind.

Beispielsweise können die katalytisch aktiven Komponenten durch Tränkung mit Lösungen oder Suspensionen der Salze oder Oxide der entsprechenden Elemente, Trocknung und anschließende Reduktion der Metallverbindungen zu den entsprechenden Metallen oder Verbindungen niederer Oxidationsstufe auf das Trägermaterial aufgebracht werden. Die zur Reduktion eingesetzten Reduktionsmittel sind vorzugsweise Wasserstoff oder komplexe Hydride.

Die katalytisch aktive Komponente kann auch auf den Träger aufgebracht werden, indem der Träger mit Lösungen thermisch leicht zersetzbarer Salze imprägniert wird und der so behandelte Träger auf Temperaturen von 300 bis 600°C erhitzt wird, wobei die adsorbierten Metallverbindungen thermisch zersetzt werden.

Thermisch leicht zersetzbare Salze sind beispielsweise Nitrate und thermisch leicht zersetzbare Komplexverbindungen wie Carbonyl- oder Hydrido-Komplexe der katalytisch aktiven Metalle.

Die thermische Zersetzung wird vorzugsweise in einer Schutzgasatmosphäre durchgeführt. Geeignete Schutzgase sind z.B. Stickstoff, Kohlendioxid, Wasserstoff oder Edelgase.

Weiterhin kann die katalytisch aktive Komponente durch Aufdampfen oder Flammspritzen auf dem Trägermaterial abgeschieden werden.

Die katalytisch aktive Komponente kann auch nach den in DE-A 25 19 817, EP-A 0 147 219 und EP-A 0 285 420 beschriebenen Verfahren auf den Träger aufgebracht werden. In den in den genannten Schriften beschriebenen Katalysatoren liegen die katalytisch aktiven Komponenten als Legierung vor, die durch Tränkung mit einem Salz oder Komplex der entsprechenden Metalle und anschließende thermische Behandlung und/oder Reduktion erzeugt werden.

Es können auch mehrere katalytisch aktive Metalle als katalytisch aktive Komponenten auf dem jeweiligen Trägermaterial aufgebracht sein.

Als Trägermaterialien können im allgemeinen die Oxide des Aluminiums und Titans, Zirkonoxid, Siliziumdioxid, Tonerden, z.B. Montmorillonite, Silikate wie Magnesiumoder Aluminium-Silikate, Zeolithe wie ZSM-5- oder ZSM-10-Zeolithe sowie Aktivkohle verwendet werden. Es können auch Mischungen verschiedener Trägermaterialien als Träger für die im erfindungsgemäßen Verfahren eingesetzten Katalysatoren dienen.

Der Gehalt der katalytisch aktiven Komponente in den Trägerkatalysatoren ist für das Gelingen des erfindungsgemäßen Verfahrens nicht kritisch. Es versteht sich für den Fachmann von selbst, daß höhere Gehalte der katalytisch aktiven Komponenten in den Trägerkatalysatoren zu höheren Raum-Zeit- Umsätzen als niedrigere Gehalte führen können Im allgemeinen beträgt der Gehalt der katalytisch aktiven Komponente im Trägerkatalysator 0,1 bis 90 Gew.-%, bevorzugt 0,5 bis 40 Gew.-%, bezogen auf die Gesamtmasse des Katalysators. Diese Angaben beziehen sich auf den gesamten Katalysator, inklusive des Trägermaterials. Dieses kann sehr unterschiedliche spezifische Dichten und spezifische Oberflächen haben, so daß diese Angaben auch unter- oder überschritten werden können, ohne daß sich dies auf das Ergebnis des erfindungsgemäßen Verfahrens nachteilig auswirkt.

Die Aktivierung der Fällungskatalysatoren und der Trägerkatalysatoren kann in situ zu Beginn der Reaktion durch den anwesenden Wasserstoff erfolgen, bevorzugt ist jedoch die separate Aktivierung dieser Katalysatoren vor ihrer Verwendung.

Beispielhaft seien die folgenden im erfindungsgemäßen Verfahren einsetzbaren heterogenen Katalysatoren genannt: Kobalt auf Aktivkohle, Kobalt auf Siliziumdioxid, Rhenium auf Aluminiumoxid, Rhenium auf Aktivkohle, Kobalt auf Aluminiumoxid, Rhenium auf Aktivkohle, Rhenium auf Siliziumdioxid, Rhenium/Zinn auf Aktivkohle, Rhenium/Platin auf Aktivkohle, Kupfer auf Aktivkohle, Kupferchromid, Bariumkupferchromid, Kupfer/Aluminium/Manganoxid, Kupfer/Aluminiumoxid/-Zinkoxid sowie Katalysatoren gemäß DE-A 39 32 332, US-A 3,449,445, EP-A 0 044 444, EP-A 0 147 219, DE-A 39 04 083, DE-A 23 21 101, EP-A 0 415 202, DE-A 23 66 264 und EP-A 0 100 406. Besonders bevorzugte Katalysatoren sind solche, die in EP-A 0 552 463 beschrieben sind.

Der Gehalt an den Wertprodukten 1,4-Butandiol, γ-Butyrolacton und THF im Hydrieraustrag kann schwanken. Er wird überwiegend durch die Parameter Druck, Temperatur und Verweilzeit und die Wahl des Hydrierkatalysators bestimmt.

Der Gehalt an γ-Butyrolacton kann bis fast auf 0 abgesenkt werden, wenn bei hohem Druck, niedriger Temperatur und mit langer Verweilzeit hydriert wird. Der Gehalt an THF ist hoch, wenn der Hydrierkatalysator saure Zentren besitzt.

Das molare Verhältnis der Wertprodukte zueinander kann beispielsweise 70 bis 99 mol% 1,4-Butandiol, 0,5 bis 29 mol-% THF und 0,1 bis 20 mol-% γ-Butyrolacton betragen, wobei die Summe der Anteile aller drei Produkte 100 mol-% ergibt.

Der Hydrieraustrag wird üblicherweise destillativ aufgetrennt. Dabei wird der gegebenenfalls im Hydrieraustrag enthaltene bei der Veresterung freigesetzte Veresterungsalkohol, sofern er noch nicht im Anschluß an die Veresterung abgetrennt wurde, zusammen mit eventuell vorhandenen Leichtsiedem wie THF zuerst vom 1,4-Butandiol abgetrennt. Ist die THF-Menge groß genug, daß sich eine Isolierung lohnt, wird das THF in bekannter Weise vom Veresterungsalkohol abgetrennt, bevor dieser in die Veresterungsstufe zurückgeführt wird.

Der 1,4-Butandiol enthaltende Produktstrom wird in dem Fachmann bekannter Weise aufgereinigt. Dabei kann γ-Butyrolacton gewonnen werden. Wenn man kein y-Butyrolacton gewinnen möchte, kann dieses problemlos in die Hydrierung zurückgeführt werden.

Gegebenenfalls im Hydrieraustrag enthaltene nicht vollständig hydrierte, gegenüber 1,4-Butandiol hochsiedenden Komponenten, wie Ester aus Butandiol und Hydroxybuttersäure oder Bernsteinsäure, lassen sich problemlos in die Hydrierung zurückführen. Das wäre bei einer Gasphasenhydrierung aufgrund der extrem geringen Flüchtigkeiten nicht möglich.

Durch das erfindungsgemäße Verfahren ist es möglich, in einem wirtschaftlichen Verfahren hochreines 1,4-Butandiol mit einer Reinheit von über 99,9 mol-%, bevorzugt über 99,95 mol-%, in hoher Ausbeute zu erhalten.

Die folgenden Beispiele beschreiben die Erfindung, insbesondere die Hydrierung in der Flüssigphase gegenüber einer Hydrierung in der Gasphase, zusätzlich.

### Beispiel 1:

In einer Reaktorkaskade umfassend Hauptreaktor (2,5 1 Kupferkatalysator, T 4489, der Süd-Chemie AG-München, Reaktor wird mit Flüssigkreislauf zur Wärmeabfuhr betrieben) und Nachreaktor (0,5 1 Kupferkatalysator, T 4489) wurden 1,5 kg/h Maleinsäuredimethylester (hergestellt aus Maleinsäureanhydrid auf Basis n-Butan durch katalytische Veresterung mit Methanol und anschließender Destillation) in Rieselfahrweise bei 250 bar in der Flüssigphase hydriert (Katalysatorbelastung 0,5 kg Diester/Liter h). Im Hauptreaktor betrug die Eingangstemperatur 150°C und die Ausgangstemperatur 208°C. Der Nachreaktor wurde bei circa 195°C betrieben. Der Umsatz an Maleinsäuredimethylester war vollständig. Die Ausbeute an 1,4-Butandiol betrug 98 mol-% (Tetrahydrofuran 1 mol-%, γ-Butyrolacton 0,4 mol-%). Als Nebenprodukt wurde n-Butanol mit 0,5 mol-% gebildet. Der Rest von 0,1 mol-% verteilte sich auf Produkte wie n-Propanol, 2-(4-Hydroxybutoxy)-Tetrahydrofuran, 2-Methoxytetrahydrofuran und Xylol. Durch fraktionierte Destillation (30 mbar) des Reaktionsaustrags konnte Butandiol mit Reinheiten von über 99,95 GC-Flächen-% erhalten werden. Die Analyse der Reaktionsausträge erfolgte gaschromatographisch mit innerem Standard, wobei als Detektor ein Flammenionisationsdetektor (FID) verwendet wurde.

### Vergleichsbeispiel:

In einem Rohrreaktor mit 800 ml Kupferkatalysator T 4489 wurde Maleinsäuredimethylester (gleiche Qualität wie im erfindungsgemäßen Beispiel) unter den Bedingungen wie in WO 97/43242 in der Gasphase hydriert
(62 bar, circa 190°C, Katalysatorbelastung 0,15 kg Diester/Liter · h). Die Ausbeuten der Wertprodukte verteilten sich wie folgt: 1,4-Butandiol 79,1 mol-%, γ-Butyrolacton 10,4 mol-%, Tetrahydrofuran 5,3 mol-%. Als Nebenprodukt entstand vor allem n-Butanol mit 4,5 mol-%. Die restlichen 0,7 mol-% verteilten sich auf Produkte wie n-Propanol, 4-Hydroxybutyraldehyd, 2-(4-Hydroxybutoxy)-Tetrahydrofuran, 2-Methoxytetrahydrofuran, Xylol, 1-Methoxy-1,4-butandiol, Butandioldiether, 4-Hydroxybuttersäuremethylester und 4-Hydroxybuttersäurebutandiolester. Die fraktionierte Destillation des Hydrieraustrags ergab maximale Butandiol-Reinheiten von nur 98,1 GC-Flächen-%. Hauptnebenkomponente war γ-Butyrolacton mit 1 GC-Flächen-%. Da sich das Butyrolacton in allen Fraktionen befand, mußte es sich Sumpf, wahrscheinlich aus 4-Hydroxybuttersäurebutandiolester, ständig nachgebildet haben. Die Analyse der Reaktionsausträge erfolgte gaschromatographisch mit innerem Standard, wobei als Detektor ein FID verwendet wurde.

## Patentansprüche

1. Verfahren zur Herstellung von 1,4-Butandiol und gegebenenfalls γ-Butyrolacton und Tetrahydrofuran durch Oxidation von n-Butan oder Benzol zu einem Maleinsäureanhydrid enthaltenden Produktstrom, Absorption von Maleinsäureanhydrid aus dem Produktstrom mit einem hochsiedenden inerten Lösungsmittel in einer Absorptionsstufe, wobei ein flüssiger Absorptionsaustrag erhalten wird, Veresterung des flüssigen Absorptionsaustrages mit einem C₁- bis C₅-Veresterungsalkohol in einer Veresterungsstufe zu einem Veresterungaustrag, der den entsprechenden Diester und hochsiedendes inertes Lösungsmittel enthält, anschließende Hydrierung des Veresterungsaustrages wobei ein Hydrieraustrag erhalten wird, der die Wertprodukte 1,4-Butandiol und optional γ-Butyrolacton und Tetrahydrofuran und den Veresterungalkohol enthält, welcher destillativ in die Wertprodukte und den Veresterungsalkohol aufgetrennt wird und der Veresterungsalkohol in die Veresterungszone zurückgeführt wird, **dadurch gekennzeichnet, daß** der Veresterungsaustrag vor der Hydrierung unter vermindertem Druck destillativ in den Diester und das inerte Lösungsmittel getrennt wird, das inerte Lösungsmittel in die Absorptionsstufe zurückgeführt wird und der Diester in der Flüssigphase an einem Festbettkatalysator hydriert wird.

2. Verfahren nach Anspruch 1, **dadurch gekennzeichnet, daß** nach der Destillation des Hydrieraustrags 1,4-Butandiol in einer Reinheit von über 99,9 mol-% erhalten wird.

3. Verfahren nach Anspruch 1 oder 2, **dadurch gekennzeichnet, daß** das zur Absorption eingesetzte inerte Lösungsmittel einen mindestens um 10°C höheren Siedepunkt als der Maleinsäurediester besitzt.

4. Verfahren nach einem der Ansprüche 1 bis 3, **dadurch gekennzeichnet, daß** als Veresterungsalkohol ein Monoalkohol aus der Gruppe Methanol, Ethanol eingesetzt wird.

5. Verfahren nach einem der Ansprüche 1 bis 4, **dadurch gekennzeichnet, daß** in der Veresterungsstufe ein homogen löslicher Katalysator als Veresterungskatalysator eingesetzt wird.

6. Verfahren nach einem der Ansprüche 1 bis 5, **dadurch gekennzeichnet, daß** die Destillation des Veresterungsaustrages bei einem Druck von 30 bis 800 mbar und einer Sumpftemperatur von 100 bis 250°C in einer Destillationskolonne durchgeführt wird.

7. Verfahren nach einem der Ansprüche 1 bis 6, **dadurch gekennzeichnet, daß** die Hydrierung bei einer Temperatur von 60 bis 300°C und einem Druck von 30 bis 330 bar durchgeführt wird.

8. Verfahren nach einem der Ansprüche 1 bis 7, **dadurch gekennzeichnet, daß** die Belastung des Hydrierkatalysators 0,2 bis 1,3 kg Diester/Liter Katalysatorvolumen · h beträgt.

9. Verfahren nach einem der Ansprüche 1 bis 8, **dadurch gekennzeichnet, daß** der Hydrierkatalysator mindestens ein Element aus der Gruppe Ib, VIb, VIIb, VIIIb, IIIa, IVa, Va des Periodensystems der Elemente enthält.

10. Verfahren nach einem der Ansprüche 1 bis 9, **dadurch gekennzeichnet, daß** das molare Verhältnis der Wertprodukte im Hydrieraustrag 70 bis 99 mol% 1,4-Butandiol, 0,5 bis 29 mol% THF und 0,1 bis 20 mol% γ-Butyrolacton beträgt, wobei die Summe der molaren Anteile von 1,4-Butandiol, THF und γ-Butyrolacton 100 mol% beträgt.

## Claims

1. A process for preparing 1,4-butanediol and, if desired, γ-butyrolactone and THF by oxidizing n-butane or benzene to form a product stream including maleic anhydride, absorbing maleic anhydride from the product stream with a high-boiling inert solvent in an absorption stage to give a liquid absorption product, esterifying the liquid absorption product with a C₁-C₅ esterifying alcohol in an esterification stage to form an esterification product comprising the corresponding diester and high-boiling inert solvent, then hydrogenating the esterification product to give a hydrogenation product which comprises the products of value, 1,4-butanediol and, if desired, γ-butyrolactone and tetrahydrofuran and the esterifying alcohol and which is separated by distillation into the products of value and the esterifying alcohol, and recycling the esterifying alcohol to the esterification zone, which comprises separating the esterification product into the diester and the inert solvent by distillation under reduced pressure prior to the hydrogenation, recycling the inert solvent to the absorption stage, and hydrogenating the diester in the liquid phase over a fixed-bed catalyst.

2. A process as claimed in claim 1, wherein following distillation of the hydrogenation product 1,4-butanediol is obtained in a purity of more than 99.9 mol%.

3. A process as claimed in claim 1 or 2, wherein the inert solvent used for absorption has a boiling point higher by at least 10°C than that of the maleic diester.

4. A process as claimed in any of claims 1 to 3, wherein the esterifying alcohol is a monoalcohol from the group consisting of methanol and ethanol.

5. A process as claimed in any of claims 1 to 4, wherein a homogeneously soluble catalyst is employed as esterification catalyst in the esterification stage.

6. A process as claimed in any of claims 1 to 5, wherein distillation of the esterification product is carried out in a distillation column at a pressure of from 30 to 800 mbar and a liquid-phase temperature of from 100 to 250°C.

7. A process as claimed in any of claims 1 to 6, wherein hydrogenation is carried out at a temperature from 60 to 300°C and at a pressure of from 30 to 330 bar.

8. A process as claimed in any of claims 1 to 7, wherein the weight-hourly space velocity of the hydrogenation catalyst is from 0.2 to 1.3 kg of diester/liter of catalyst volume . h.

9. A process as claimed in any of claims 1 to 8, wherein the hydrogenation catalyst comprises at least one element from groups Ib, VIb, VIIb, VIIIb, IIIa, IVa and Va of the Periodic Table of the Elements.

10. A process as claimed in any of claims 1 to 9, wherein the molar proportion of the products of value in the hydrogenation product is from 70 to 99 mol% 1,4-butanediol, from 0.5 to 29 mol% THF and from 0.1 to 20 mol% γ-butyrolactone, the sum of the molar fractions of 1,4-butanediol, THF and γ-butyrolactone being 100 mol%.

## Revendications

1. Procédé pour la préparation de 1,4-butanediol et éventuellement de γ-butyrolactone et de tétrahydrofuranne, au moyen d'une oxydation du n-butane ou du benzène en un courant de produits contenant de l'anhydride maléique, au moyen d'une absorption de l'anhydride maléique, à partir du courant de produits, à l'aide d'un solvant inerte à haut point d'ébullition, dans une étape d'absorption dans laquelle on obtient un courant d'absorption liquide, au moyen d'une estérification du courant d'absorption liquide, à l'aide d'un alcool d'estérification en C₁ à C₅, dans une étape d'estérification pour obtenir un courant d'estérification qui contient le diester correspondant et un solvant inerte à haut point d'ébullition, et au moyen d'une hydrogénation ultérieure du courant d'estérification dans laquelle on obtient un courant d'hydrogénation contenant, en tant que produits de valeur, le 1,4-butanediol et éventuellement la γ-butyrolactone ainsi que le tétrahydrofuranne, ainsi que l'alcool d'estérification que l'on sépare, par distillation, en produits de valeur et en alcool d'estérification, l'alcool d'estérification étant recyclé dans la zone d'estérification, **caractérisé en ce que**, avant l'hydrogénation, le courant d'estérification est séparé, par distillation, sous pression réduite, en diester et en solvant inerte, le solvant inerte est recyclé dans l'étape d'absorption, et le diester est hydrogéné en phase liquide en présence d'un catalyseur à lit fixe.

2. Procédé selon la revendication 1, **caractérisé en ce que** l'on obtient, après la distillation du courant d'hydrogénation, le 1,4-butanediol présentant une pureté supérieure à 99,9% en moles.

3. Procédé selon la revendication 1 ou 2, **caractérisé en ce que** le solvant inerte mis en oeuvre pour l'absorption présente un point d'ébullition qui est supérieur d'au moins 10°C à celui du diester maléique.

4. Procédé selon l'une quelconque des revendications 1 à 3, **caractérisé en ce que** l'on met en oeuvre, en tant qu'alcool d'estérification, un monoalcool du groupe formé par le méthanol et l'éthanol.

5. Procédé selon l'une quelconque des revendications 1 à 4, **caractérisé en ce que** l'on met en oeuvre, dans l'étape d'estérification, en tant que catalyseur d'estérification, un catalyseur soluble de façon homogène.

6. Procédé selon l'une quelconque des revendications 1 à 5, **caractérisé en ce que** l'on réalise la distillation du courant d'estérification dans une colonne de distillation, sous une pression allant de 30 mbars à 800 mbars et à une température de fond allant de 100°C à 250°C.

7. Procédé selon l'une quelconque des revendications 1 à 6, **caractérisé en ce que** l'on réalise l'hydrogénation à une température allant de 60°C à 300°C et sous une pression allant de 30 bars à 330 bars.

8. Procédé selon l'une quelconque des revendications 1 à 7, **caractérisé en ce que** la charge du catalyseur d'hydrogénation va de 0,2kg à 1,3 kg de diester/litre de volume du catalyseur · h.

9. Procédé selon l'une quelconque des revendications 1 à 8, **caractérisé en ce que** le catalyseur d'hydrogénation contient au moins un élément groupe Ib, VIb, VIIb, VIIIb, IIIa, IVa, Va de la Classification Périodique des Eléments.

10. Procédé selon l'une quelconque des revendications 1 à 9, **caractérisé en ce que** le rapport molaire des produits de valeur contenus dans le courant d'hydrogénation va de 70% à 99% en moles pour le 1,4-butanediol, de 0,5% à 29% en moles pour le THF, et de 0,1% à 20% en moles pour la γ-butyrolactone, la somme des proportions molaires du 1,4-butanediol, du THF et de la γ-butyrolactone étant égale à 100% en moles.
